# EUROPEAN PATENT APPLICATION

(11) **EP 2 990 972 A1**
(43) Date of publication of application: **02.03.2016**
(21) Application number: 13881117.9
(22) Date of filing: 02.04.2013
(51) Int. Cl.: G06F 19/00, A61B 1/00

(54) **TELEMEDICINE SYSTEM FOR REMOTE CONSULTATION, DIAGNOSIS AND MEDICAL TREATMENT SERVICES**

(71) Applicant: Espinosa Escalona, Fernando Pablo José, 01020 Distrito Federal (MX); Iglesias Ramos, Carlos Guillermo, 03100 Distrito Federal (MX); Morales Medel, Alan, 01219 Distrito Federal (MX)
(72) Inventor: Espinosa Escalona, Fernando Pablo José, 01020 Distrito Federal (MX); Iglesias Ramos, Carlos Guillermo, 03100 Distrito Federal (MX); Morales Medel, Alan, 01219 Distrito Federal (MX)
(74) Representative: ABG Patentes, S.L.
(86) International application number: PCT/MX2013/000039
(87) International publication number: WO 2014/163475

(57) **Abstract**

The invention relates to a telemedicine system including improved methods, systems and techniques for providing remote consultation, diagnosis and/or medical treatment. Said system uses a Basic Telemedicine Unit BTU and Specialist Telemedicine Unit STU comprising the simultaneous and independent display, on the local interface and on the remote interface, of all of the patient-related audio, video, biomedical data signals and clinical data, the concurrent annotation of images and videos, and processing for the protection of the personal data of the patients.

## Description

### FIELD OF INVENTION

The field of invention is Telemedicine, in particular, the systems, methods and techniques to be used by health care professionals, preferably within medical facilities, to provide remote consultations, diagnosis, and medical assistance.

The core of the process is the exchange of clinical and biomedical patient information between two or more telemedicine workstations. It consists of the independent and simultaneous display of information in each workstation, using Information and Communication Technology. The information is stored in two sections; one contains clinical information and the other the personal information of the patient. It is stored in a database after encrypted.

### BACKGROUND OF THE INVENTION

The World Health Organization (WHO) states that e-Health is the use of Information and Communication Technology (ICT) in healthcare services. E-Health units work in conjunction with each other on a global, national, and regional level. They promote and strengthen the use of ICT in the healthcare industry, both in the field and in an administrative sense. The eHealth unit at WHO is based in the Department of Knowledge, Ethics and Research in the cluster of Health Systems and Innovation.

The American Telemedicine Association (ATA) defines telemedicine as the use of medical information exchanged between different sites using electronic communication to generally improve a patient's health. Along with telemedicine, the term, "Tele-health" is used in a broader sense and may or may not include clinical services, e-health stations for patients, remote monitoring of vital signs, continuing medical education, and call-centers for nurses.

The WHO has pinpointed the following advantages to telemedicine:
a) It reduces the waiting period for a patient to receive medical attention by a licensed healthcare professional;
b) It reduces the cost of medical treatment by diminishing travel expenses for patients to locations that offer the specialized care they require;
c) It increases the access to healthcare services by connecting a wide variety of specialists that can each speak to their particular area of expertise. The amount of healthcare professionals is only limited to how many of them are found in any given location that possesses a telemedicine unit;
d) It improves the perception of the type of healthcare that is provided in remote communities that do not always have access to specialists, when said communities are given access to these networks.

Telemedicine services are varied and serve a variety of purposes.

There are systems to monitor patients at home, other systems monitor patients at a healthcare facility, for instance, patients in a hospital or in an intensive care unit.

Certain systems provide remote diagnosis and medical assistance services. To do so, they transmit audio, video and biomedical data. These systems aim to connect two or more healthcare professionals within specialized facilities so that a service may be provided.

Patients that live in remote or isolated communities may receive better healthcare services. The attention they receive can be more frequent and/or more specialized. This directly improves the quality of the healthcare services provided and benefits the health conditions of the inhabitants of said community.

Another advantage to telemedicine is that it allows severe medical conditions to be treated quicker, hopefully avoiding patient deaths or prolonged periods of rehabilitation. The different benefits of prompt treatment -within 60 minutes in a personal or virtual form- by a healthcare professional is widely acknowledged in the medical community.

Of the different telemedicine systems currently in existence, patents and patent applications have been issued that show the current State of the Art.

An example of a telemedicine system is the one found in U.S. patent No. 5,987,519 filed by Peifer and collaborators, granted on November 16 1999. It describes a telemedicine service that captures data, video, and audio, which is later retrieved, in order to exchange medical information between a central monitoring hub and a remote patient portal. The system of said patent states that: 1) The Telemedicine System based on data transmission units sent between a central hub and a remote patient portal that can be exchanged through different networks; 2) A variety of biomedical devices are connected to the remote patient portal, which then relays the information it collects to the central control unit; 3) The patient monitoring unit also includes videoconference interface that operates with another videoconference interface to exchange audio and video; 4) The information captured in the remote unit and retrieved in the central unit; 5) The control unit in the central monitoring station displays clinical information, and videoconference information, and processes each of them separately.

The telemedicine service described in said patent is limited to a monitoring system for individual patients that require a patient monitoring unit in their homes; it is not thereby designed to provide remote consultation, diagnosis and medical assistance between different healthcare professionals to multiple patients with the same telemedicine unit.

Likewise, even though said monitoring system connects different biomedical devices so that patient information may be sent to the central monitoring unit, it is limited in the sense that it has to select the source of information and does not allow for the simultaneous display of audio and video.

This monitoring system does not allow annotations to be made on the medical images exchanged between the patient monitoring unit and the central hub, thereby hindering consultation and diagnosis services.

Another example of a telemedicine system is the one found in the Patent Application Publication US 2011/0178373 by Pacey and collaborators, published July 21^{st} 2011, which describes a telemedicine system which consists in: 1) A method to operate a Telemedicine Base Unit connects to a remote attention site; 2) The transmission of a signal generated by a piece of medical equipment to the remote unit; 3) The reception, in the base unit, issues instructions for the operation of the piece of medical equipment from the remote unit, in accordance with the information that was originally transmitted; 4) The instructions provide a guide to operate a piece of medical equipment identified as the signal generator; 5) The method also comprises the transmission of audio and video from the telemedicine unit to the remote assistance unit; 6) Patient medical data is also transmitted to the remote site; 7) Reception of graphic instructions from the remote site which are annotated over the signal generated by the connected piece of medical equipment. Annotations over sent graphic instructions.

The system and methods of telemedicine described in the referred patent application publication presents a fundamental disadvantage in the sense that it is a system and a methodology that is used exclusively to provide remote instructions in the use of a piece of medical equipment connected to a telemedicine unit by a specialist located in a central hub using video, audio and data generated by a remote unit. It is not designed to provide consultation, diagnosis, and/or medical assistance by health care professionals. The invention description states that the described unit may be used with a variety of biomedical devices, but it does not show that it may be used by several biomedical devices in a simultaneous fashion. The design of this invention is clearly limited to a sole unit seeing as the system operates automatically once the single piece of medical equipment is connected.

Another example of an antecedent of a telemedicine system and methodology to provide remote e-Health services is the one found in the Publication of U.S. Patent Application US 2012/0179479 by Waterson and collaborators published July 12^{th} 2012 that describes Systems and Methodologies for Remote E-Health Services which consists in: 1) A system to provide remote medical consultation between a Medical Booth and a remote Medical Call Center (MCC); 2) The medical booth is able to place medical equipment on a patient in response to instructions issued remotely by a healthcare professional; 3) The MCC can graphically display patient data or information; 4) Two-way communication between the MCC and the Booth, including transmitting data and videoconference.

The system mentioned above is limited for diagnostic and medical assistance purposes because it was designed for a patient to enter a Booth in which he or she may have access to medical equipment that will relay his information to a central station. The booth or kiosk consists of a spot that recollects biomedical data in order to be analyzed by a central hub. It does not handle images and data in a simultaneous fashion and it does not have an annotation system that allows the patient and healthcare specialist to interact and ultimately provide a diagnosis.

Considering the current state of this field of knowledge, it would be advantageous if a telemedicine system existed that was designed to provide consultation, diagnosis and medical treatment service to patients, using information and telecommunication technologies that allow simultaneous and independent display of information in different work stations. A telemedicine service designed for these purposes, would enable specialized physicians located in highly specialized or better equipped medical facilities to aid healthcare professionals located in different areas, with consultation, diagnosis and medical assistance.

This technique allows healthcare specialists located in different locations to, using telemedicine equipment, simultaneously display and transmit all medical information of a patient through audio, video and data from any piece of medical equipment connected to the telemedicine unit, which is used to obtain medical information of the patient, yielded from a consultation. This can be used in any field of medicine, including cardiovascular concerns, dermatology, respiratory system, etc.

Likewise, the telemedicine units should transmit and display the medical history of a patient and enable videoconference with units operated by specialists.

It would be convenient for healthcare specialists to be able to receive and make use of, independently of any actions carried out by healthcare professionals elsewhere, all information including data, video, and audio, generated by all biomedical devices connected to the telemedicine unit of the patient; to generate annotations and give visual indications over the data and video to the remote unit; to have access to all the information contained in the medical history; and along with a videoconference, to carry out remote medical consultation in order to reach a diagnosis and propose a form of treatment for the patient.

### BRIEF DESCRIPTION OF THE DRAWINGS

Figure 1 is a block diagram of the Telemedicine System of the invention described herein.
Figure 2 is a block diagram of an example of a Telemedicine Operation System found in the memory/processing module of the Basic Telemedicine Unit (BTU.)
Figure 3 is a flowchart that shows a method to carry out the multiplication of audio and video signals from biomedical devices by creating and managing device suppliers. The multiplied signals may be displayed independently in the BTU and STU.
Figure 4 is a flowchart that shows how audio and video signals are combined and/or grouped to the device suppliers. The added video signals are integrated to a sole video channel and the mixed audio signals are added to a sole audio channel.
Figure 5 is a flowchart of the processing of audio, video, and data signals from biomedical devices connected to the BTU.
Figure 6 is a flowchart that displays how information related to the remote consultation, diagnosis and medical assistance is generated and stored in the designated storage units designated by this invention in order to protect any personal information of the patient.

### DETAILED DESCRIPTION OF THE PREFERRED EMBODIMENT OF THE INVENTION

In the following description, certain specific details are included to provide a thorough understanding of the various embodiments of the invention. However, for any person knowledgeable in this art will understand that the invention may be practiced without these details or with various combinations of these details. The specific forms of the invention described below represent improved methods, systems and techniques to provide remote consultation, diagnosis, and/or medical assistance in real-time as well as in a delayed mode (by first storing and then transmitting information).

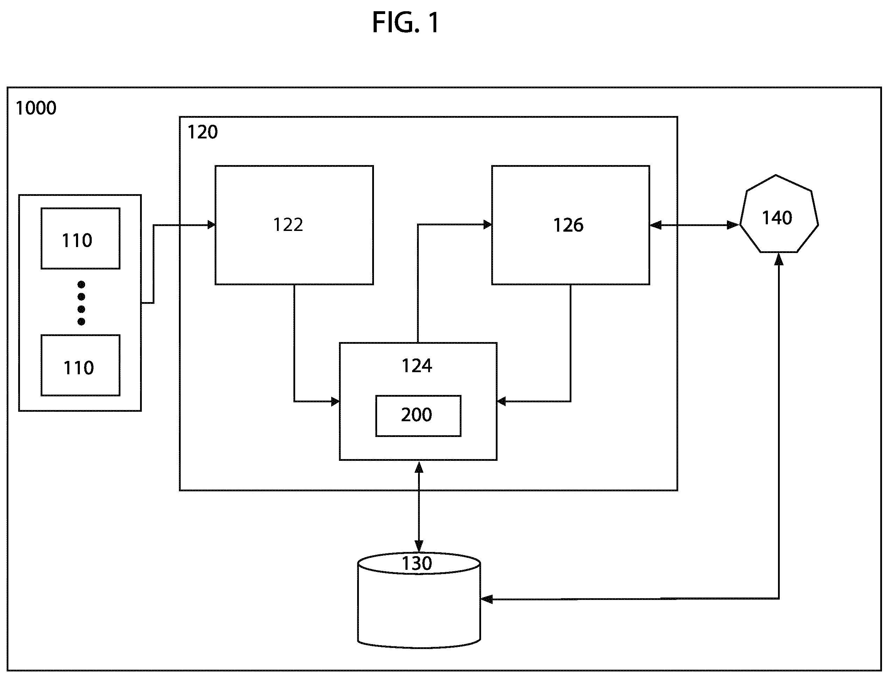

Figure 1 is an example of a block diagram of a particular Telemedicine System (1000) comprising a basic telemedicine unit (120) (BTU); one or more biomedical devices (110) connected to the BTU that provide patient medical data; one or more specialized telemedicine units (140) (STU) consisting of devices capable of transmitting and receiving audio/video and data either directly or indirectly to or from the BTU; and one or more systems (130.) for storage, encryption, and data protection

The BTU (120) may be a device (e.g. computer, laptop, smartphone and/or any device that can connect in a wired or wireless network) from which you can set up a multidirectional communication (e.g., audio, video, graphic and/or any other indication of content). The BTU has Entry System Devices (122) a Memory/Processing Module (124) based on software and/or hardware and a Communication System (126.)

The Processing module (124) has a built-in Telemedicine Operation System (200) that allows any medical data of a patient generated by any biomedical devices connected to the BTU to be transmitted to a STU in which a medical specialist performs a remote consultation to develop a diagnosis and propose a treatment for patients located in a remote location through the Specialized Telemedicine Unit (STU). To perform specialist medical consultation STU has access simultaneously and independently access to all information of biomedical devices currently connected to the Basic Telemedicine Unit.

The Telemedicine system (200) includes a configuration module system (210), a communications administration system (220) that sends and receives date between the BTU and STU, and a user interface (230). The system configuration module (210) comprises an audio, video, and data capture system (212), a data generator system (214) that multiplies and groups the signals issued by the biomedical devices, which are simultaneously displayed in the user interface and processed in the communication management system (220) in order to be sent to the specialized telemedicine unit (140).

The biomedical devices (110) that provide patient medical information can be audio type (e.g. line level, microphone level, digital stereo and monaural) video (e.g. SECAM, PAL, NTSC ATSC type CV, YC, RGB, RGBHV), and data (e.g. image files, JPEG, JPEG 2000, BMP, GIF, RAW, audio WMA, MP3, AIFF, video MPEG, MPEG-2, MPEG-4, WMV, chains and/or flows serial/parallel data).

The BTU connects to the STU through a telecommunications network that may be comprised by different mediums (e.g. twisted pair, coaxial, fiber optics, radio electric frequency) and communication protocol (e.g., TCP/IP, Ethernet, Wi-Fi, WiMAX) with remote medical facilities.

The BTU includes a signal input device (122) that may consist of different connectors according to input signal type, video devices can be connected through various connectors and/or wired media and/or wireless (e.g. RCA, S-Video, coaxial, DVI, DB-15, HDMI, DisplayPort, components, Bluetooth, Wi-Fi, USB 2.0, USB 3.0), audio devices be connected through various connectors and/or wired media and/or wireless (e.g. RCA mono, RCA stereo, coaxial, TRS 3.5mm, TRRS 3.5mm, HDMI, Bluetooth, Wi-Fi, USB 2.0, USB 3.0), data devices can be connected through various connectors and/or wireline and / or wireless means (e.g. ZigBee, Bluetooth, Wi-Fi, USB 2.0, USB 3.0, RS-232).

The memory/processing module (124) of the BTU that can be implemented through software based on different programming techniques such as object-oriented (e.g. Java, C++, C#, Visual Basic, .NET), procedure-oriented (e.g. C, Pascal, Ada, Modula), script and/or declaratory and/or through hardware (e.g. cards DAQ data acquisition, integrated audio/video systems, PLCs, RAM) and a system Communication (126) which may be based on software and/or hardware (e.g. routers, switches, bridges, hubs, firewalls, NICs).

The Telemedicine Operating System (200) provides multiple logical (e.g. code, instructions, routines, etc.) and/or services connected with the operation of the Basic Telemedicine Unit (120), and provides instructions that, when executed, receives the information and data generated by the biomedical devices (110). This information may be audio type (e.g. line level, microphone level, digital stereo, and monaural) video (e.g. SECAM, PAL, NTSC ATSC type CV, YC, RGB, RGBHV), and data (e.g. image files, JPEG, JPEG 2000, BMP, GIF, RAW, audio WMA, MP3, AIFF, video MPEG, MPEG-2, MPEG-4, WMV, chains and / or flows of serial/parallel data).

The Telemedicine Operating System (200) multiplies the audio and video signals and manages a multi-directional communication with the STU (140). The Telemedicine system (200) can provide other functions such as access to stored video and/or stored audio, connecting to other medical monitoring devices, as wired or wireless, and/or other administrative tasks related to the implementation of the conditions around the telemedicine system (1000).

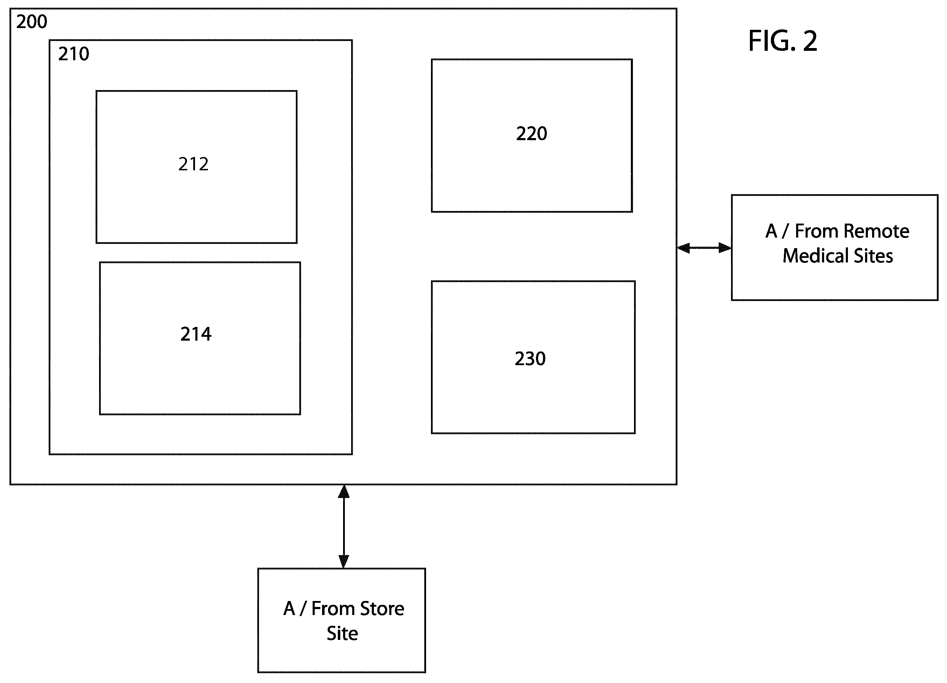

Figure 2 is a block diagram containing the components of an example of a Telemedicine System (200). In a particular application, the Telemedicine System (200) comprises one or more functional components/modules that work together to allow multidirectional communication with Specialized Telemedicine Units (140) and for receiving signals generated by biomedical devices (110). The components can be integrated through software, hardware, or a combination of both.

The Telemedicine Operation System (200) comprises a system configuration module (210), a communication management system (220), and a user interface (230). The system configuration module (210) includes an audio, video, and data capture system (212) for receiving and filtering the information of connected biomedical devices (110) and a Data Manager System (214) programmed for the multiplication and simultaneous handling of all the signals of the devices connected to the Basic Telemedicine Unit.

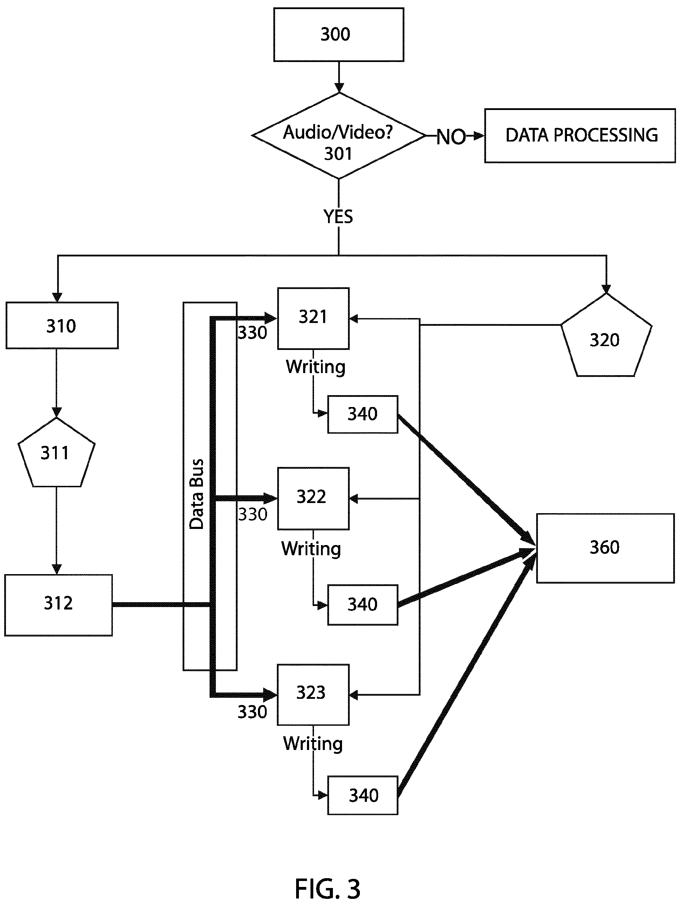

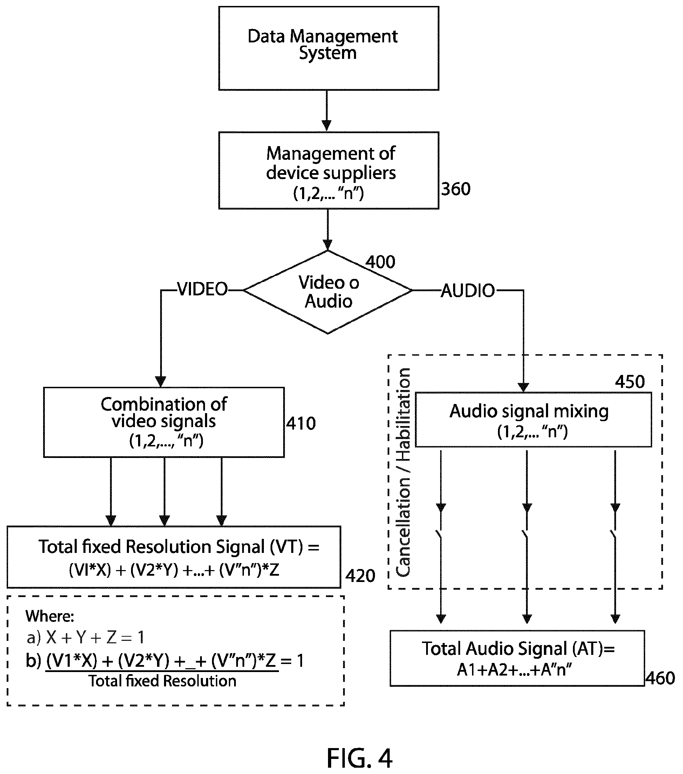

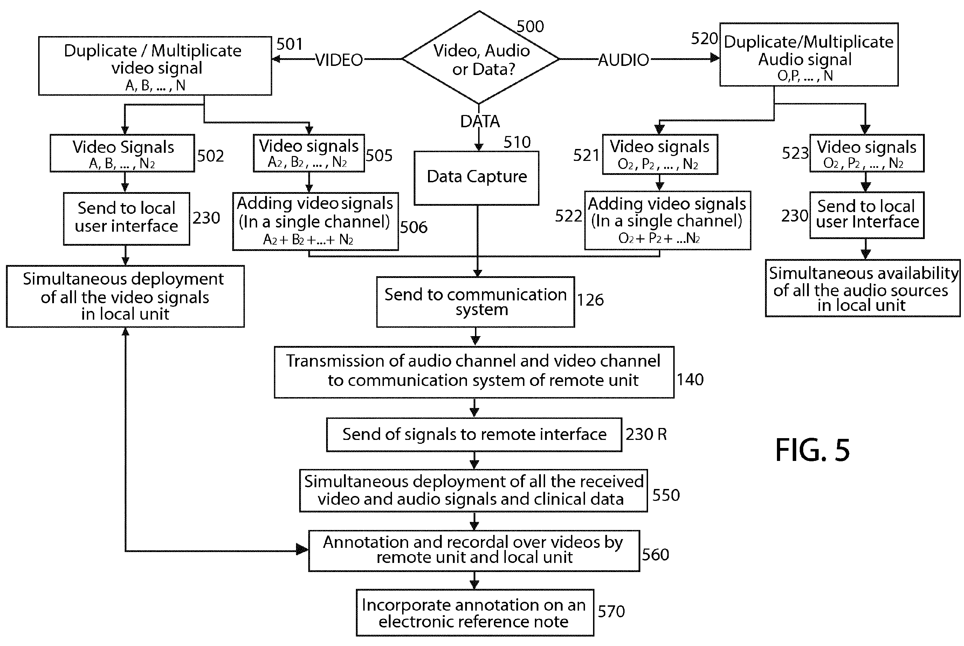

The communication management system (220) is programmed to transmit (e.g. forward, send, communicate, etc.) the signals generated by connected biomedical devices (110) and various input signals related to the BTU (120) to the different remote attention sites STU (140) and for receiving from the STU, audio, video, data, and annotations made on videos that facilitate the remote medical consultation. A communication, in one of the specific arrangements, may include for example audio/video/graphic indications from the remote STU (140). Communication from the remote medical site can, for example, include instructions on how to place a medical device on a patient in order to get a read out. The Telemedicine System (200) includes user interface (230) for simultaneously displaying the information of biomedical devices and/or receive instructions via annotated images and/or videos from the STU (140) as to how to collect information from the patient. The system configuration module (210) includes a filter to capture audio, video and data (212) to determine the type of signal received, a signal generation system (214) that multiplies the audio and video signals and groups them into a single channel. The method to handle the signals received by the system is displayed graphically in the multiplication flowcharts of the signal received in Figure 3, in the integration flowcharts of the signals available in figure 4, and in the information-processing flowchart of the biomedical devices of Figure 5, that incorporates the aforementioned multiplication and integration methods.

Figure 3 is a flow chart illustrating the method by means of which the signals issued by the biomedical devices multiply once the Audio, Video, and Data Capture System (212) catalogues and filters the type of signal received by the system (300), its function may be based on software and/or hardware (e.g. NICs, audio/video selectors, audio/video matrixes, capture cards, computer algorithms). The system (212) determines the S memory demand (310) that requires a connected video signal in order to store an information box, the amount of storage space required by the box will depend on the resolution and the frames per second contained in the processed signal (greater resolution will require greater space). After determining the amount of memory required, the system (212) will write a unique memory block (311) of S size, the information of one frame of video is discarded and rewritten at the original frequency contained in the signal (e.g. 25fps, 30fps, 59fps, 60fps,), single memory block enabled (312) can be simultaneously read by any device provider, a device provider being the piece of hardware and/or software in charge of reading the memory card for the post-processing of the signal (for example, to display in: LCD monitor, CRT, Plasma, LED, GUI, software based visualizer, graphics). The system (212) also determines the demand of memory required by an audio signal that was received so a segment of information may be stored, the amount of memory space required by the segment will depend on the resolution of the processed signal (greater resolution will require greater space, for example, 4kHz, 16kHz, 48kHz, 96kHz), after determining the amount of memory required, the system (212) is in charge of writing in a single block of memory of an audio segment which is discarded and rewritten at the frequency of the received signal (e.g. 20Kbps, 64Kbps, 132Kbps, 352Kbps). The unique habilitated memory block can be simultaneously read by any device provider, a device provider being the piece of hardware and/or software in charge of reading the memory card for the post-processing of the signal (for example, analogue speakers, digital speakers, systems, cards, and audio processors). The system (212) temporarily stores the data obtained from the biomedical devices in a memory location that is available for read/write timeless way for any resource (e.g., manipulating, transmitting, copying, deleting) and deployment in the user Interface (230).

As shown in figure 3, the system (212) Capture Audio, Video, and Data by filtering a signal (300) received in audio and/or video form, it interacts with the Data Manager System to create and/or manage the device suppliers depending on system demands (321), (322), (323). A device provider is created when a video and/or audio signal must be multiplied. This device provider has the capacity to request to the Audio, Video, and Data Capture System (212) all information found in the requested unique memory block (audio or video). The system (212) shifts the content of the unique memory block to the S-sized memory block (312) to the Data Manager System (214) by means of a data-bus connection (e.g., serialized, parallel) to be later read by a device provider (330) that may have an additional memory or S size storage unit to record the content read in memories 1.2, "n" (340) for the post-processing of the signal. This procedure allows the information in one audio/video signal to be read multiple times without blocking reading/writing capabilities of the original signal and to be independently processed as many times as required by the device suppliers. The amount of device suppliers will depend on the amount of memory resources, and the processing capacity available in Module (124). Said amount may be increased by physical means (for example, processors, RAM, data storage units).

Figure 4 is a flow chart showing the method as audio and video signals are grouped. The Data Manager System (214) manages the information of the Device suppliers (360). If the signal (400) to be managed is a video signal (410), the Data Manager System (214) combines all video signals (410) stemming from device suppliers into a single signal combined with fixed resolution (420) (e.g., 720p, 1080p, 4K) distributing them equally within the signal. The Data Manager System (214) allows the individual stored resolution within a device provider to be increase and decreased to the maximum level of the combined video signal reducing the size of all other signals of device suppliers using the relation (420) shown in Figure 4. If the signal (400) to be administered is an audio signal (450), the Data Manager System (214) groups the audio signals stemming from the different device suppliers into a solo, mixed, audio signal (460). The Data Manager System (214) allows the audio information (450) stored in the device provider to be cancel or activated, hence allowing for the individual muting of audio signals in order to give user the option to hear what suits him.

The processing of information flow of the biomedical devices is shown in Figure 5. The Audio, Video, and Data Capture System (212), shown in figure 2, detects the type of signal received (500). A received video signal is duplicated (501) by the Data Manager System (214) as described in Figure 4. The first duplicated image (502) is sent to the local user interface (230) and is deployed concurrently with all other signals received at the user device. Another duplicated image (505) flow through the method described in relation to Figure 3, is grouped with other duplicated images (506) to be sent to the communication system (220) for independent delivery and deployment in the STU.

The audio, video, and data capture system (212) in figure 2 detects if audio signals are received. A received audio signal is duplicated (520) by the Data Manager System (214) as is described in Figure 4. The first duplicated audio (523) is sent to the user interface (230) and may be heard in the local unit. The second duplicated audio (521) is grouped with the other duplicated audio signals (522) to be sent to the communications administration system (220). This replication process may be repeated as often as required depending on the demand of the user and the system.

The Communication management system (220) (CMS) receives the combined video signal through a physical connection (e.g., HDMI, RCA, DVI, VGA), the mixed audio signal through a physical connection (e.g., HDMI, RCA, 3.5mm TRS), and the data compiled during the consult via a data bus connection (i.e., serial, parallel). The CMS (220) transmits the received signals depending on their nature (i.e., audio, video, or data) to the User Interface (230), the Communication System (126), and the Database (130). Signal combined video generated is sent to the user interface (230) by a physical connection (such as HDMI, RCA, DVI, VGA) to the communication system (126) where it is processed and converted to one of the video transmission standards (e.g., H.263, H.263+, H.264, H.265) and be sent to remote STU stations (140) by distributed transmission techniques (e.g., TCP/IP sockets, RTP). Signal mixed audio generated is sent to the user interface (230) by a physical connection (e.g., HDMI, RCA, 3.5mm TRS) and to the communication system (126) where it is processed and converted to one of the audio transmission standards (e.g., G.711, G.722, G.722.1, 64 kbps MPEG4 AAC-LD) and be sent to remote stations (140) using distributed transmission techniques (e.g., TCP/IP sockets, RTP). The number of signals transmitted, audio and video, will depend on the number of remote connection sites, the minimum amount being 2 video signals and 2 audio signals for a point to point connection (local + remote) and maximum "n" for multipoint connections (local + remote 1 + remote2 +...+ remote "n"). When the information to be transmitted is exclusively data (e.g., text files, image files, video files, and audio files) the CMS (220) establishes a data connection (e.g., TCP/IP, RMI, HTTP, web services: XML, AJAX, SOAP) with the server where the database (130) is found and transmits the information in a secure form (e.g., 64bits, 128bits) for its encryption and storage.

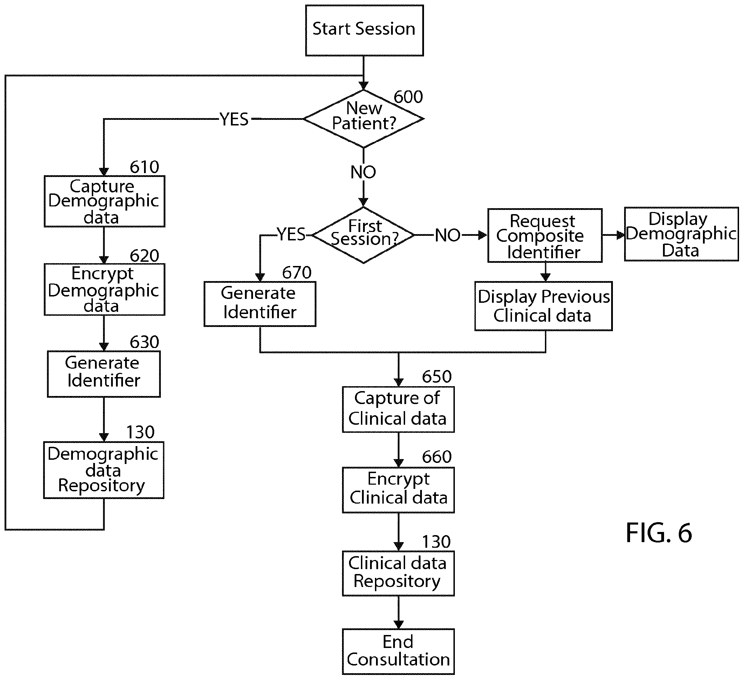

The Telemedicine service described herein has an encrypted information storage method in a storage unit (130) as shown in Figure 1 that may be the arrangement of one or several software and hardware elements. The storage encryption pattern is based on two centralized and independent storage units whose information management method is shown in the Flowchart contained in Figure 6.

The flow starts with determining (600) if the capture of the patient demographic of the patient is required. If yes, being a new patient, the system displays in the user interface (230) a format to capture demographic data (610), and the healthcare professional enters the required data. Once the patient demographic is captured, it is encrypted (620) and sent to the repository which have been previously provided, either a repository-based hardware or repository based software location, for this purpose database (130) in figure 1. The system generates an identifier (630) that contains the information required to retrieve the patient information previously captured. It is redetermined (600) if patient demographic data is required or if a patient identifier already exists. If a patient identifier (630) already exists, all information linked to the identifier and the existing patient is then displayed, if it does not then a screen (650) is displayed wherein the patient's chart may be captured. All signals received from the diverse biomedical devices (110) are simultaneously displayed in the BTU interface (230). All images, sounds and data from the biomedical devices that were used during the consult are recorded 660 in the format 650 if selected, along with any notes made by the healthcare professionals. Once the consult has finished, all clinical information is encrypted and sent to the repository, which have been previously provided, either a repository-based or repository-based software or a combination of both (130). The system generates an indicator that contains all information necessary to retrieve the clinical information (670) that was just stored and is added to the identifier of patient demographic information (630). The identifier made up of both identifiers (630) and (670) is stored in a different storage location to the storage location that contains patient demographics and clinical information, previously used, either a repository-based or repository-based software or a combination of both (130).

The preferred embodiment of the invention, as described herein, comprises a Telemedicine Unit (120), as shown in Figure 1, where its components include a Device Entry System for Multiple Biomedical devices (122) with one or more digital HDMI audio/video connectors, at least two S-type Video/RCA analogue video connectors, at least two RCA stereo analogue audio connectors, at least one 3.5mm TRS audio analogue connector, some USB 2.0 and 2 USB 3.0 connection ports, an internal Bluetooth antenna and a Wi-Fi antenna (802.11 a/b/g/n.) The system is designed to preferentially be connected to -without this limiting its capacity to connect to other devices- the following: a digital stethoscope with Bluetooth capabilities, a vital signs monitor with 802.11 Wi-Fi, an EKG machine with USB 2.0 capabilities, a digital dermatoscope reader with YC and S-Video ports, a digital otoscope with CV and RCA entry ports and an ultrasound port with HDMI/DVI port. It has a Memory/Processing module (124) preferably with 6 GB RAM DDR3a 1600MHZ memory expandable for future use and with a processor of at least 2.4 GHZ and 32nm technology. The system configuration module (210) runs on a Windows 7 or an operating system with 64 bits or more, although it should be stated that any other operating system might be installed. The Audio, Video, and Data capture system (212) as well as the Data Manager System (214) are preferably hardware based, although not in a limiting sense, in audio/video digital/analogue acquisition cards, and as far as software goes, preferably, although not limited to, .NET 4.5. or more advanced programming tools running on a WPF graphic subsystem. The storage units required are taken depending on the administration of the RAM or of preferably solid storage units, for example, with a 60 GB minimum capacity. A communication system (126) with Fast Ethernet 10/100 Mbps ports, routing capacity and functions, VPN creation and access lists, NAT, DHCP and IPsec technology that can send and receive, through TCP/IP packaging, all kinds of signals stemming from the Communication and Administration System (220), this system (220) is preferably based on hardware in an IP telephone device and SIP protocol, a teleconference device in a Telepresence version with communications capacities in SIP and H.323 protocols for the pre-packaging and conversion standards for the transmission of AAC-LD audio/H.264 video, NIC's for the TCP/IP pre-packaging of generated information and creation of peer-to-peer channels and independent audio/video streaming. The user interface (230) is displayed in a touch-sensitive monitor with a minimum of 10 simultaneous touches capacity; the screen may be any size although it should preferably be no smaller than 27" with a minimum resolution of 1920 x 1080 points and 60 frames per second.

The storage units of the Telemedicine Unit (120), in this preferred mode, are external to the BTU and the STU that interact in the remote consultation, diagnosis, and medical assistance, and may be found indistinctly in a central storage unit with a RAID 5 system and with a SWL preferential server database created for the storage of additional services and future applications in the cloud service that allow the consultation and analysis of all information regardless of Physical Units.

In accordance with the preferred Telemedicine Operation System (200) found in Figure 2, it is the one that with an Audio, Video, and Data Capture system carries out the multiplication and grouping of audio and video signals that are simultaneously and independently displayed in the user interface of the BTU (230), characterized by displaying in a single screen the information received from biomedical devices, clinical patient information and the images and audio from videoconferences, which is handled by the user through the touch screen mentioned above and whose signals are multiplied and then sent to an independently handled STU, through one of the screens with medical-grade resolution and a minimum size of 20", 1080p resolution, and DICOM tone control, and a Tele-presence screen with a minimum size of 32", 1080p resolution for a constant display of the patient.

Most preferably, the system of the present invention is characterized by comprising:
- a basic telemedicine unit BTU (120) that includes an entry system for medical devices (122), a memory/processing module (124) based on software and/or hardware, and a communication system (126) to process, convert, transmit, and receive audio, video, and data signals to and from one or several remote stations:
- at least one specialized telemedicine unit STU (140), in direct communication with the basic telemedicine unit, which has the necessary capacity to indistinctly send and receive audio, video, and data, directly or indirectly, to and from a basic telemedicine unit;
- one or several biomedical devices (110) connected to the BTU destined to collect information regarding the patients' medical condition; and
- one or several storage, encryption, and patient information protection mediums (130), in direct communication with the basic telemedicine unit and with the remote telemedicine unit;
- wherein the BTU and the STU allow the simultaneous and independent display, in a local and a remote interface, of all audio, video, and biomedical data and clinical data of the patient, the concurrent annotation over images and video and treatment for the protection of personal data of the patient.

Meanwhile, in one preferred embodiment, the method for providing remote consultation services, medical diagnosis and treatment to patients, which is the exchange of clinical and biomedical patient information between two or more telemedicine workstations, by plugging in biomedical devices to a basic telemedicine unit and using information and communication technology, is comprised of the following stages:
I. Multiplying the audio and video signals stemming from biomedical devices to a basic telemedicine unit, by:
   a) receiving the audio or video signal from a medical device;
   b) determining the amount of S memory required by the audio or video signal received in order to be stored in a segment of information or information box; said memory demand of the segment will depend on the resolution of the received signal;
   c) recording the received signal in a new S-sized memory block;
   d) creating device suppliers;
   e) reading the new S-sized memory block for each generated device provider and recording the information in memory spaces linked to each provider; and
II. grouping the audio and video signals of the connected biomedical devices, in accordance with the following:
   a) once all signals have been duplicated, separating and classifying them into audio and video signals through a device provider administration system;
   b) adjusting the resolution of the video signals so the total sum of all resolution equals the maximum available resolution of the user interface; and
   c) allowing the user to decide which signals to mute so the total audio signal will be the sum of all non-muted signals;
and wherein the information of the audio and video signals are simultaneously and independently displayed in the user interface and in the diverse BTU and STU units.

Although the invention has been described in the context of the preferred embodiment or preferential mode, to those skilled in the art will be clear that the scope of the concept exemplified extends beyond the system architecture and method described and illustrated specifically to other potential alternative forms of embodiment of the invention that are feasible or viable. Furthermore, although the invention has been described in detail, one skilled in the art to which the invention pertains may deduct that some components of the system and/or method steps can be substituted or added that are different from those described, without modifying essentially the result for which they are intended.

Giving the foregoing, it is intended that the scope of the present invention is not interpreted as limited by the particular embodiment described, but this is determined by a reasonable interpretation of the contents of the following claims.

## Claims

1. A telemedicine system to provide remote consultation, diagnosis, and/or medical treatment services, comprising:
- a basic telemedicine unit BTU (120) that includes an entry system for biomedical devices (122), a memory/processing module (124) based on software and/or hardware, and a communication system (126) to process, convert, transmit, and receive audio, video, and data signals to and from one or more remote stations;
- at least one specialized telemedicine unit STU (140), in flow communication with the basic telemedicine unit, which has the necessary capacity to indistinctly send and receive audio, video, and data, directly or indirectly, to and from a basic telemedicine unit;
- one or more medical devices (110) connected to the BTU for collecting information about the medical condition of the patient; and
- one or more means for storage, encryption, and protection of patient information systems (130), in flow communication with the basic telemedicine unit and with the remote telemedicine unit;
wherein the BTU and the STU allow simultaneous and independent deployment, in both local interface and remote interface, all audio, video, and biomedical data and clinical data of a patient, concurrent annotation on images and video and the proper treatment for the protection of personal data of the patient.

2. The system of claim 1, wherein the module (124) memory/processor comprises a telemedicine operation system (200) that includes:
- a module (210) with an audio, video, and data capture system (212) to receive and filter information from biomedical devices to the Basic Telemedicine Unit BTU, which detects the type of signal received, and with a data management system (214) duly configured for the multiplication and simultaneous handling of all signals generated by biomedical devices into a single channel;
- a communication management system (220) configured to send and receive data between the BTU and the STU through the communication system (126); said delivery system transmits signals generated by biomedical devices connected (110) and various input signals related to the BTU (120) to the remote STU sites of care (140), and to receive from the STU, audio, video, and data signals related to the remote medical consultation process; and
- a user interface (230);

3. The system of claim 1, wherein the signals of the biomedical devices (110), connected to the basic telemedicine unit BTU (120), are displayed simultaneously in the user interface (230) and processed in the communication management system (220) so that they may be sent to the STU specialized telemedicine unit (140).

4. The system of claim 1, wherein the audio, video, and data capture system (212) determines the amount of S memory (310) required by a received audio, video, or data signal to be stored in an information box, and is in charge of writing a unique S-sized memory block (311) of the information box, which is discarded and rewritten in the original frequency of the signal.

5. The system of claim 4, wherein the audio, video, and data capture system (212), when it filters a received audio and/or video signal (300), interacts with the data management system to create "n" device suppliers (320).

6. The system of claim 2, wherein the data management system (214) is responsible for creating and/or manage the device suppliers depending on system demand.

7. The system of claim 1, wherein the information received from an information signal may be read multiple times without blocking the reading/writing capabilities of the original signal and to be independently processed as many times as necessary by the device suppliers.

8. The system of claim 6, wherein the data management system (214) manages the information of the device suppliers (360) such that if the managed signal is a video signal, said data management system (214) combines all video signals from the device suppliers into a single video signal with fixed resolution, distributing them equally within said signal.

9. The system of claim 6, wherein the data management system (214) manages the information of the device suppliers (360) so that if the managed signal is an audio signal, said data management system (214) combines all audio signals from the device suppliers into a single mixed audio signal.

10. The system of claim 6, wherein the data management system (214) is configured to remove or enable the stored audio information in a device provider, allowing the user to mute individual audio signals and enable the user to hear what suits him.

11. The system of claim 2, wherein the communication management system (220) transmits the received signals, depending on their nature, to the user interface (230), to the communications system (126) and the database (130).

12. The system of claim 1, wherein the communication system (126) is configured to send and receive any signal from the communication management system (220).

13. A telemedicine system to provide remote consultation, diagnosis, and medical assistance services to patients, comprising:
- a basic telemedicine unit **BTU** (120) that includes an entry system for biomedical devices (122), a memory/processing module (124) based on software and/or hardware, and a communication system (126) to process, convert, transmit, and receive audio, video, and data signals to and from one or more remote stations:
- at least one specialized telemedicine unit **STU** (140), in flow communication with the basic telemedicine unit, which has the necessary capacity to indistinctly send and receive audio, video, and data, directly or indirectly, to or from the basic telemedicine unit;
- one or more biomedical devices (110) connected to the BTU intended to gather information about the medical condition of the patient; and
- one or more means for storage, encryption, and protection of patient information (130), in flow communication with the basic telemedicine unit and with the remote telemedicine unit; wherein the memory/process module (124) comprises a telemedicine operation system (200) that includes:
- a module (210) with an audio, video, and data capture system (212) to receive and filter information from biomedical devices to the Basic Telemedicine Unit BTU, which detects the type of signal received, and with a data management system (214) duly configured for the multiplication and simultaneous handling of all signals from biomedical devices into a single channel;
- a communication management system (220) configured to send and receive information between the BTU and the STU through the communication system (126); said delivery system transmits the signals generated by the connected biomedical devices (110) and various input signals related to the BTU (120) to the remote STU sites of care (140), and to receive from the STU, audio, video, and data signals and annotations on video to facilitate the process of medical consultation from a patient at a distance; and
- a user interface (230); and
wherein the BTU and the STU allow simultaneous and independent deployment in both local interface and remote interface, all audio, video, and biomedical data and clinical data of a patient, concurrent annotation on images and video and treatment for the protection of personal data of the patient.

14. The system of claim 13, wherein the signals of the biomedical device (110), connected to the basic telemedicine unit BTU (120), are displayed simultaneously in the user interface (230) and processed in the communication management system (220) so that they may be sent to the STU specialized telemedicine unit (140).

15. The system of claim 13, wherein the audio, video, and data capture system (212) determines the memory demand S (310) required by a received audio, video, or data signal to be stored in an information box, and is responsible of writing a unique S-sized memory block (311) of the information box, which is discarded and rewritten in the original frequency of the signal.

16. The system of claim 13, wherein the audio, video, and data capture system (212), when it filters a received audio and/or video signal (300), interacts with the data management system to create "n" device suppliers (320).

17. The system of claim 13, wherein the data management system (214) is responsible for creating and/or administrating the device suppliers depending on system demands.

18. The system of claim 13, wherein the information received from an information signal may be read multiple times without blocking the reading/writing capabilities of the original signal and be processed independently and as often as necessary by the device suppliers.

19. The system of claim 13, wherein the data management system (214) manages the information of the device suppliers (360) such that if the signal to be managed is a video signal, said data management system (214) combines all video signals from the device suppliers into a single video signal with fixed resolution, distributing them equally within said signal.

20. The system of claim 13, wherein the data management system (214) manages the information of the device suppliers (360) so that if the managed signal is an audio signal, said data management system (214) combines all audio signals from the device suppliers into a single mixed audio signal.

21. The system of claim 13, wherein the data management system (214) is configured to remove or enable the stored audio information of a device provider, allowing the user to mute individual audio signals to enable the user to hear what suits him.

22. The system of claim 13, wherein the communication management system (220) transmits the received signals, depending on their nature, to the user interface (230), to the communications system (126) and the database (130).

23. The system of claim 13, wherein the communication system (126) is configured to send and receive any signal from the communication management system (220).

24. A method for providing remote consultation services, medical diagnosis and treatment to patients, which consists in the exchange of clinical and biomedical patient information between two or more telemedicine workstations, plugging in a medical device to a unit or basic telemedicine unit using information and communication technology; said method is comprised of the following steps:
I. Multiplying the audio and video signals from biomedical devices to a basic telemedicine unit, through:
a) entering the audio or video signal of a medical device;
b) determining the demand of memory S required by the audio or video signal received in order to be stored in a segment of information or information box; said memory demand of the segment will depend on the resolution of the processed signal;
c) recording the signal received at a new memory block size S;
d) creating device suppliers;
e) reading the new memory block of size S for each generated device provider and record the information in memory spaces linked to each provider; and
II. grouping the audio and video signals of the connected biomedical devices, as follows:
a) once all signals have been duplicated, separating and classifying them into audio and video signals through a device provider management system;
b) adjusting the resolution of the video signals so that the total sum of all resolution equals the maximum available resolution of the user interface; and
c) allowing the user to decide which signals to mute so the total audio signal will be the sum of all non-muted signals;
and wherein the information of audio and video signals is displayed simultaneously and independently in the user interface and the various BTU and STU stations.

25. The method of claim 24, further comprising preserving and protecting the patient personal information through the following steps:
a. capturing, encrypting, and recording the patient demographic information in an external repository to the basic telemedicine unit, generating a demographic identifier;
b. capturing, encrypting, and recording clinical patient information in an external repository to the basic telemedicine unit, independent of the repository that stores the demographic information, generating a clinical identifier; and
c. integrating a composite identifier comprised of the demographic identifier and the clinical identifier, and recording information of the composite identifier in a repository separate to that of the demographic information and the clinical information.

26. The method of claim 24, wherein the data input of biomedical devices in the system are temporarily stored in a memory location that is available for read/write timeless way for any resource such, handling, transfer, copy, deletion and display in the user interface.

27. The method of claim 24, in which device suppliers are created and/or managed depending on the system demand.

28. The method of claim 24, which allows information from an audio/video signal to be read multiple times without blocking the reading/writing capabilities of the original signal and to be processed independently as many times as necessary by the device suppliers.

29. The method of claim 24, wherein the information of an information signal can be read multiple times without blocking the reading/writing capabilities of the original signal and be processed independently and as often as necessary by the device suppliers.

30. The method of claim 24, wherein all multiplied video signals are grouped into a single video channel and all multiplied audio signals are mixed in a single audio channel for transmission to the remote telemedicine units STU.

31. The method of claim 24, which allows concurrent annotations on any video and/or image displayed in the user interface, which is visible from the units or the interconnected telemedicine stations.

32. The method of claim 24, wherein the security of personal patient data are preserved by storing, encrypting and separating his or her information to a third independent means of storage that is independent from the basic telemedicine unit and the specialized telemedicine unit.
